(19) Europäisches Patentamt
European Patent Office
Office européen des brevets

(11) Veröffentlichungsnummer: **0 318 423 B1**

# (12) EUROPÄISCHE PATENTSCHRIFT

(45) Veröffentlichungstag der Patentschrift: **01.02.95**

(51) Int. Cl.6: **C07D 239/26**, C07D 239/32, C07D 237/10, C07D 317/10, C07D 303/48, C07D 213/30, C07D 285/12, C09K 19/34

(21) Anmeldenummer: **88710048.5**

(22) Anmeldetag: **22.11.88**

Die Akte enthält technische Angaben, die nach dem Eingang der Anmeldung eingereicht wurden und die nicht in dieser Patentschrift enthalten sind.

## (54) Flüssigkristalline Cyclopropyl-alkyl- oder -alkenyl-Heterocyclen, Verfahren zu ihrer Herstellung und ihre Verwendung in flüssig-kristallinen Mischungen.

(30) Priorität: **25.11.87 DE 3739884**

(43) Veröffentlichungstag der Anmeldung:
**31.05.89 Patentblatt 89/22**

(45) Bekanntmachung des Hinweises auf die Patenterteilung:
**01.02.95 Patentblatt 95/05**

(84) Benannte Vertragsstaaten:
**AT BE CH DE FR GB IT LI LU NL SE**

(56) Entgegenhaltungen:
**EP-A- 0 244 129**

(73) Patentinhaber: **HOECHST AKTIENGESELLSCHAFT**

**D-65926 Frankfurt (DE)**

(72) Erfinder: **De Meijere, Armin, Prof. Dr.**
**Vor den Hockenkuhlen 26**
**D-2105 Seevetal (DE)**
Erfinder: **Dübal, Hans-Rolf, Dr.**
**Heuhohlweg 6**
**D-6240 Königstein/Taunus (DE)**
Erfinder: **Escher, Claus, Dr.**
**Amselweg 3**
**D-6109 Mühltal (DE)**
Erfinder: **Hemmerling, Wolfgang, Dr.**
**Billtalstrasse 32**
**D-6231 Sulzbach (Taunus) (DE)**
Erfinder: **Müller, Ingrid, Dr.**
**Am Pfingstbrunnen 1**
**D-6238 Hofheim am Taunus (DE)**
Erfinder: **Ohlendorf, Dieter, Dr.**
**Am Kühlen Grund 4**
**D-6237 Liederbach (DE)**
Erfinder: **Wingen, Rainer, Dr.**
**Rotkäppchenweg 10**
**D-6234 Hattersheim am Main (DE)**

EP 0 318 423 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die ungewöhnliche Kombination von anisotropem und fluidem Verhalten der Flüssigkristalle hat zu ihrer Verwendung in einer Vielzahl von elektro-optischen Schalt- und Anzeigevorrichtungen geführt. Dabei können ihre elektrischen, magnetischen, elastischen und/oder thermischen Eigenschaften zu Orientierungsänderungen benutzt werden. Optische Effekte lassen sich dann beispielsweise mit Hilfe der Doppelbrechung, der Einlagerung dichroitisch absorbierender Farbstoffmoleküle ("guest-host mode") oder der Lichtstreuung erzielen.

Zur Erfüllung der ständig steigenden Praxisanforderungen auf den verschiedenen Anwendungsgebieten besteht laufend ein Bedarf an neuen verbesserten Flüssigkristall("liquid crystal")-Mischungen und somit auch an einer Vielzahl mesogener Verbindungen unterschiedlichster Struktur. Dies gilt sowohl für die Gebiete, bei denen nematische LC-Phasen (z. B. TN = "twisted nematic", STN = "supertwisted nematic", SBE = "supertwisted birefringence effect", ECB = "electrically controlled birefringence") verwendet werden, als auch für solche mit smektrischen LC-Phasen (z. B. ferroelektrische, elektrokline).

Viele der für LC-Mischungen geeigneten Verbindungen lassen sich durch ein Aufbauprinzip (Bauschema) beschreiben [siehe z. B. J. Am. Chem. Soc. 108, 4736 (1986), Struktur I; Science 231, 350 (1986), Fig. 1 A; J. Am. Chem. Soc. 108, 5210 (1986), Fig. 3], bei dem Kerne aus cyclischen Verbindungen - Aromaten, Heteroaromaten, aber auch gesättigte Ringsysteme - mit geradkettigen oder in der Kette durch kleine Gruppen (z. B. Methyl, Chlor) substituierten und somit verzweigten Alkylseitenketten verknüpft sind.

Verbindungen, die eine terminal-cyclopropyl-substituierte Alkylkette als Teilstrukturelement aufweisen, sind z. B. aus den US-A 3 948 961, 3 966 969 und 4 014 922 (Henrick et al.) bekannt. Die Verbindungen sollen als Insektizide geeignet sein, über flüssigkristallines Verhalten wird nicht berichtet. Die in diesen Veröffentlichungen aufgeführten Verbindungen unterscheiden sich von den nachfolgend definierten insbesondere dadurch, daß sie mit dem Molekülrest immer über eine Esterfunktion verknüpft sind und in diesem Molekülrest zwar aromatische, aber keine heteroaromatischen Ringsysteme aufweisen.

In der EP-A 0 224 129 werden 2,2-Dimethylcyclopropan-Derivate beschrieben, die optisch-aktiv sind und über eine -CO-O-$CH_2$-, -O-CO- oder -$CH_2$-Brücke (am rechten Ende des jeweiligen Brückenglieds befindet sich der Cyclopropylring) mit einem der bekannten mesogenen Reste verbunden sind. Diese Verbindungen sollen als Komponenten für ferroelektrische LC-Mischungen (spontane Polarisation bis zu 11 $nC/cm^2$) geeignet sein.

Aufgabe der vorliegenden Erfindung ist es, neue mesogene Verbindungen zur Verfügung zu stellen, die mit vielen anderen Komponenten zu unterschiedlichsten LC-Mischungen kombiniert werden können. Die nachstehend definierten Verbindungen lösen diese Aufgabe:
Flüssigkristalline Cyclopropylalkyl- oder -alkenyl-Heterocyclen der allgemeinen Formel (I)

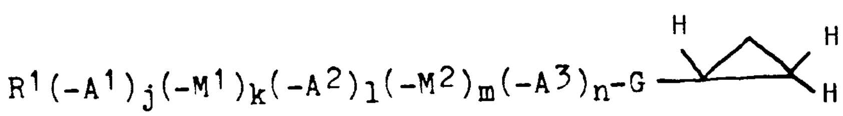

(I)

in der bedeuten:

R¹ geradkettiges oder verzweigtes (mit oder ohne asymmetrisches C-Atom) Alkyl oder Alkenyl mit 2 bis 16-C-Atomen, wobei auch eine oder zwei nicht-benachbarte -$CH_2$-Gruppen durch -O-, -S-, -CO-, -CO-O-, -O-CO- oder -O-CO-O- ersetzt sein können und wobei auch H durch F ersetzt sein kann,
oder einer der nachfolgenden Reste

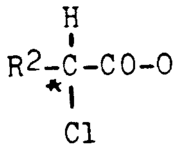 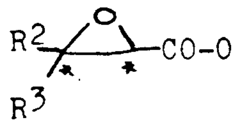 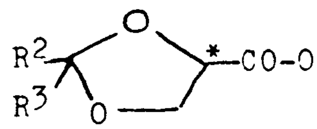

3

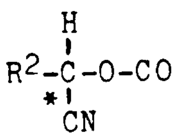 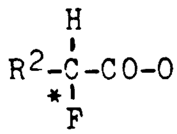 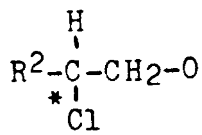

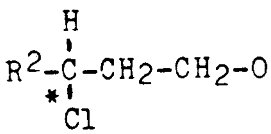 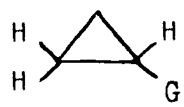

| | |
|---|---|
| $A^1,A^2,A^3$ | gleich oder verschieden 1,4-Phenylen, trans-1,4-Cyclohexylen, Pyrazin-2,5-diyl, Pyridazin-3,6-diyl, Pyridin-2,5-diyl, Pyrimidin-2,5-diyl, (1,3,4)-Thiadiazol-2,5-diyl |
| $M^1,M^2$ | gleich oder verschieden CO-O, O-CO, CO-S, S-CO, $CH_2$-O, O-$CH_2$ |
| G | geradkettiges oder verzweigtes Alkylen mit 1 bis 16 C-Atomen oder Alkenylen mit 2 bis 16 C-Atomen, bei dem auch eine oder zwei nicht-benachbarte -$CH_2$-Gruppen durch -O-, -S-, -O-CO-, -CO-O-, -S-CO- oder -CO-S- ersetzt sein können |
| $R^2,R^3,R^4$ | H oder geradkettiges oder verzweigtes Alkyl mit 1 bis 16 oder Alkenyl mit 2 bis 16 C-Atomen, bei dem auch eine -$CH_2$-Gruppe durch -O-, -CO-O- oder -O-CO- ersetzt sein kann |
| j,k,l,m,n | Null oder 1 |

mit folgenden Maßgaben: a) $j + l + n = 2$ oder 3, b) eine der Gruppierungen $A^1$, $A^2$, $A^3$ ist nicht 1,4-Phenylen oder trans-1,4-Cyclohexylen.

Bevorzugt sind solche Verbindungen, bei denen in der allgemeinen Formel (I) die Gruppierung $(-A^1)_j(-M^1)_k(-A^2)_l(-M^2)_m(-A^3)_n$- bedeutet:

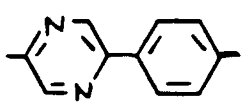, 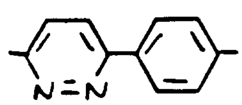, 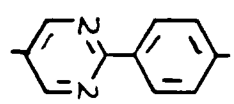, 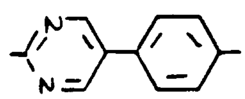,

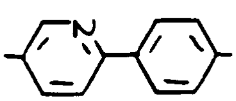, 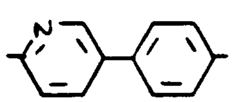, 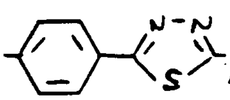, 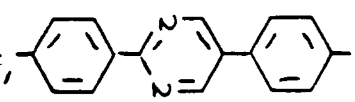,

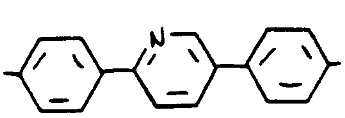, 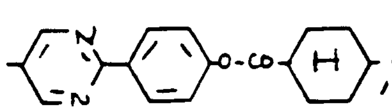, 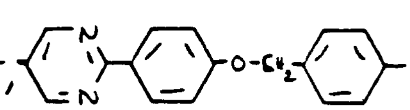,

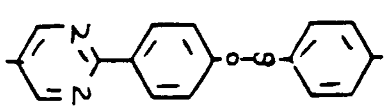, 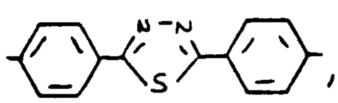, 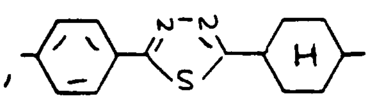,

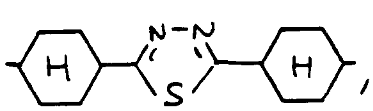, 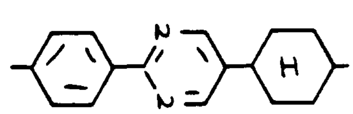, 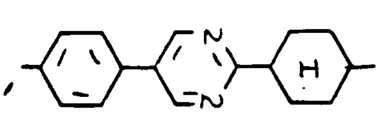

Die neuen Cyclopropylalkyl- oder -alkenyl-Heterocyclen sind chemisch, photochemisch und thermisch stabil und verfügen über eine gute Mischungskompatibilität. Im Vergleich zu den entsprechenden n-Alkyl-Homologen besitzen sie oftmals einen niedrigeren Wert für die optische Anisotropie Δn und führen in Mischungen häufig zu niedrigeren Schmelzpunkten.

Eine weitere Lösung der gestellten Aufgabe ist eine beispiels weise nematische oder smektische Flüssigkristall-Mischung mit einem Gehalt an mindestens einer flüssigkristallinen Verbindung, die als flüssigkristalline Verbindung mindestens eine Verbindung der allgemeinen Formel (I) enthält.

Die Flüssigkristall-Mischungen bestehen aus 2 bis 20, vorzugsweise 2 bis 15 Komponenten, darunter mindestens einer der erfindungsgemäß beanspruchten Verbindungen. Die anderen Bestandteile werden vorzugsweise ausgewählt aus den bekannten Verbindungen mit nematischen, cholesterischen und/oder geneigt-smektischen Phasen, dazu gehören beispielsweise Schiffsche Basen, Biphenyle, Terphenyle, Phenylcyclohexane, Cyclohexylbiphenyle, Pyrimidine, Zimtsäureester, Cholesterinester, verschieden überbrückte, terminal-polare mehrkernige Ester von p-Alkylbenzoesäuren. Im allgemeinen liegen die im Handel erhältlichen Flüssigkristall-Mischungen bereits vor der Zugabe der erfindungsgemäßen Verbindung(en) als Gemische verschiedenster Komponenten vor, von denen mindestens eine mesogen ist, d. h. als Verbindung, in derivatisierter Form oder im Gemisch mit bestimmten Cokomponenten eine Flüssigkristall-Phase zeigt [ = mindestens eine enantiotrope (Klärtemperatur > Schmelztemperatur) der monotrope (Klärtemperatur < Schmelztemperatur) Mesophasenbildung erwarten läßt].

Von der oder den erfindungsgemäßen Verbindung(en) enthalten die Flüssigkristall-Mischungen im allgemeinen 0,01 bis 70 Gew.-%, insbesondere 0,05 bis 50 Gew.-%.

Die erfindungsgemäßen Heterocyclen können nach an sich bekannten Standardreaktionen aus mesogenen monofunktionell-reaktionsfähigen Grundkörpern durch Verknüpfung mit ebenfalls monofunktionell-reaktionsfähigen Cyclopropylalkyl-Verbindungen hergestellt werden, wobei die Synthese beider Komponenten als bekannt vorausgesetzt werden kann.

Elektrooptische Bauteile, die Flüssigkristallmischungen gemäß Patentanspruch 7 enthalten, sind ebenfalls Gegenstand der vorliegenden Erfindung.

So können beispielsweise mesogene Hydroxy- oder Mercapto-Verbindungen mit Cyclopropyl-alkanolen in Anwesenheit von Triphenylphosphin/Azodicarbonsäurediester (Mitsunobu-Reaktion, z. B. in J. Chem. Soc. Perkin Trans. 1975, 461) verknüpft werden. Es können auch die separat oder intermediär erzeugten Alkali- oder Erdalkalisalze dieser mesogenen Hydroxy- oder Mercapto-Verbindungen mit Halogen-, Toluolsulfonyloxy- oder Methylsulfonyloxy-cyclopropylalkyl-Verbindungen umgesetzt werden (Williamson-Reaktion, z. B. in Patai, The Chemistry of the Ether Linkage, Interscience Publishers, New York 1967, S. 446 - 468).

Es können aber auch mesogene Carbonsäuren mit Cyclopropylalkanolen unter Kondensationsbedingungen (z. B. in March, Advanced Organic Chemistry, 2nd Ed., Mc. Graw-Hill Kogakuska Ltd., Tokyo 1977, S. 363 - 365) umgesetzt werden. In gleicher Weise ist dies auch mit mesogenen Hydroxy- oder Mercapto-Verbindungen und Cyclopropyl-alkansäuren möglich.

Die zur Verknüpfung erforderlichen Cyclopropylalkyl-Verbindungen werden nach Standardmethoden hergestellt, es wird dazu auf die vorstehend erwähnten Veröffentlichungen (US-A) von Henrick et al verwiesen.

In den nachfolgenden Beispielen verhalten sich Gew.-Teile zu Vol.-Teilen wie kg zu 1.

**Beispiel 1**

5-Heptyloxy-2-[4-(9-cyclopropyl-nonyl)oxy-phenyl]-pyrimidin

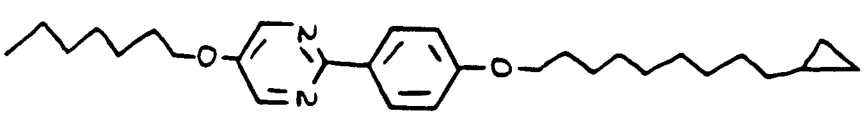

Zur Lösung von 0,52 Vol.-Teilen Diethylazodicarboxylat und 0,85 Gew.-Teilen Triphenylphosphin in 20 Vol.-Teilen Tetrahydrofuran werden 0,5 Gew.-Teile 9-Cyclopropylnonanol und 0,95 Gew.-Teile 4-(5-Heptyloxypyrimidin-2-yl)-phenol gegeben. Nach einer Reaktionszeit von 24 h wird das Lösemittel abdestilliert und der Rückstand chromatographisch ($SiO_2/CH_2Cl_2$) gereinigt. Nach Umkristallisation aus 2-Propanol werden 0,52 Gew.-Teile farblose Kristalle erhalten.
Phasenfolge: K 56,4 $S_c$ 71,3 $S_A$ 83,4 N 85,1 I

**Beispiel 2**

5-Hexyl-2-[4-(9-cyclopropyl-nonyl)oxy-phenyl]pyrimidin

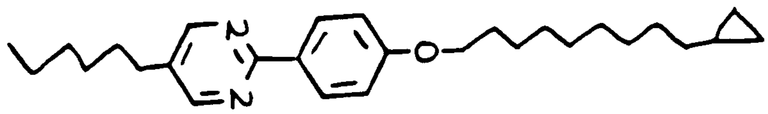

Die nachfolgenden Synthesen werden nach den Angaben des Beispiels 1 mit entsprechender Mengenanpassung durchgeführt.
Phasenfolge: K 44 N 53 I

**Beispiel 3**

5-Octyl-2-[4-(9-cyclopropyl-nonyl)oxy-phenyl]pyrimidin

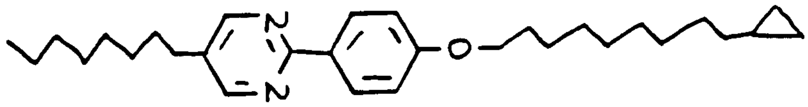

Phasenfolge: K 41,3 $S_c$ 51 $S_A$ 57,6 N 60,2 I
n ∥ 1,616 n ⊥ 1,486 Δn = 0,13 (bei 45 °C, 589 nm)
Meßmethode: Eine wichtige Kenngröße für die Güte des Kontrastes eines LC-Displays ist die optische Doppelbrechung Δn = n∥ - n⊥. n∥ bzw. n⊥ sind die Brechungsindices für parallel bzw. senkrecht zum Direktor n polarisiertes Licht. Beide Brechungsindices sind temperatur- und wellenlängenabhängig mit dem Abbé-Refraktometer bestimmbar.

**Beispiel 4**

5-Octyloxy-2-[4-(9-cyclopropyl-nonyl)oxy-phenyl]pyrimidin

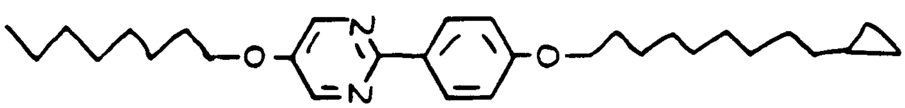

Phasenfolge: K 69,2 $S_c$ 75,8 $S_A$ 90,2 I

**Beispiel 5**

5-Nonyl-2-[4-(9-cyclopropyl-nonyl)oxy-phenyl]pyrimidin

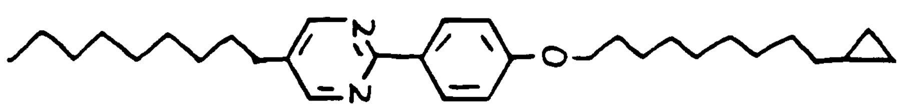

Phasenfolge: K 52,8 $S_c$ 56,8 $S_A$ 67,2 I

**Beispiel 6**

5-Decyl-2-[4-(9-cyclopropyl-nonyl)oxy-phenyl]pyrimidin

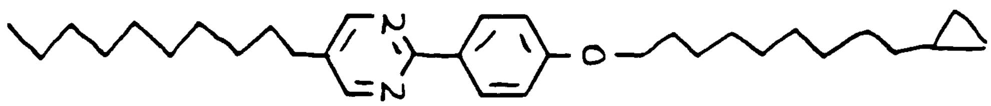

Phasenfolge: K 44 $S_c$ 64,9 $S_A$ 67,7 I

**Beispiel 7**

5-Undecyl-2-[4-(9-cyclopropyl-nonyl)oxy-phenyl]pyrimidin

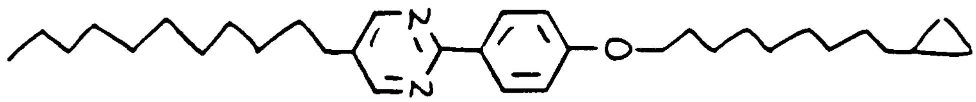

Phasenfolge: K 48 $S_c$ 70,2 $S_A$ 71,8 I

**Beispiel 8**

5-Undecyloxy-2-[4-(9-cyclopropyl-nonyl)oxy-phenyl]-pyrimidin

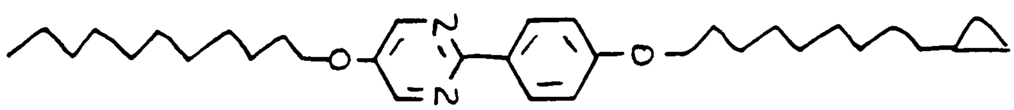

Phasenfolge: K 68 $S_c$ 95 I

**Beispiel 9**

5-Dodecyl-2-[4-(9-cyclopropyl-nonyl)oxy-phenyl]pyrimidin

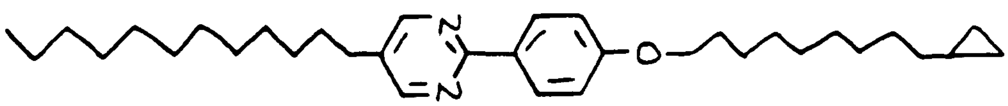

Phasenfolge: K 52 $S_c$ 72,3 I

**Beispiel 10**

2-Decylthio-5-[4-(9-cyclopropyl-nonyl)oxy-phenyl]pyrimidin

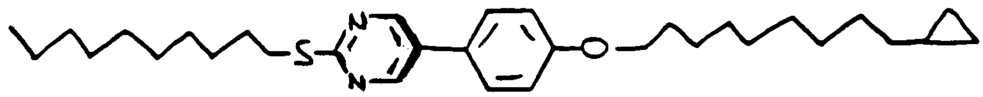

Phasenfolge: K 67,5 [38 $S_2$ 57 $S_c$ 65] I

**Beispiel 11**

5-(9-Cyclopropyl-nonyl)oxy-2-(4-heptyloxy-phenyl)pyrimidin

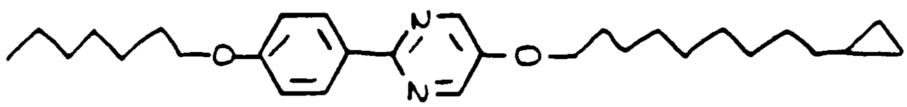

Phasenfolge: K 64,7 $S_c$ 91 I

**Beispiel 12**

5-(9-Cyclopropyl-nonyl)oxy-2-(4-octyloxy-phenyl)pyrimidin

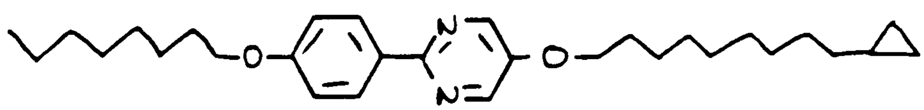

Phasenfolge: K 63,7 $S_c$ 93,2 I

**Beispiel 13** (Vergleichsbeispiel)

5-Octyl-2-[4-⟨5-((3S)-2,2-dimethylcyclopropyl)-3-methylpentyl⟩oxy-phenyl]pyrimidin

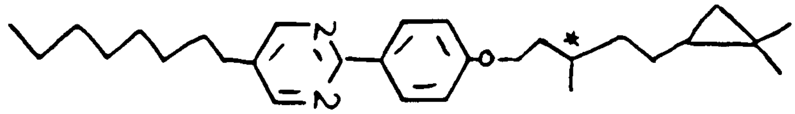

Phasenfolge: K [-21,5 $S_A$ 16] 18,5 I
$[\alpha]_D^{25}$ : -5,2 (c = 5, $CH_2Cl_2$)

Meßmethode: Versetzt man ein (nicht-chirales) Lösemittel mit einer kleinen Menge einer chiralen Verbindung, so wird die Ebene des linear polarisierten Lichts um den (charakteristischen) Winkel $\alpha$ gedreht; dieser Winkel wird wie folgt angegeben: $[\alpha]^T_D$(c = x, LM), wobei die Symbole folgende Bedeutung haben : x = Konzentration der Lösung in g/l, LM = Lösemittel, D = 589 nm (NaD-Linie), T = Temperatur der Lösung. Der Drehwinkel wird in einem Polarimeter nach 10 cm Durchgang des Lichts bestimmt.

**Beispiel 14**

(2S,3S)-4-[5-(9-Cyclopropyl-nonyl)oxy-pyrimidin-2-yl]-phenyl-2-chlor-3-methyl-pentansäureester

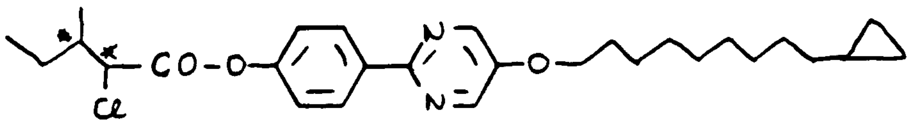

Phasenfolge: K [38 $S_c$ 40 $S_A$ 44,3] 48,5 I
$[\alpha]_D^{25}$ = -2,0 (c = 4, $CH_2Cl_2$)

**Beispiel 15**

5-Octyl-2-[4-(6-cyclopropyl-hexyl)oxy-phenyl]pyrimidin

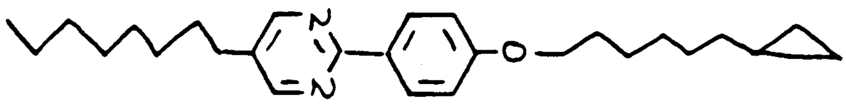

Phasenfolge: K 39 $S_c$ 46 $S_A$ 50 N 59,5 I

**Beispiel 16**

5-Decyl-2-[4-(6-cyclopropyl-hexyl)oxy-phenyl]pyrimidin

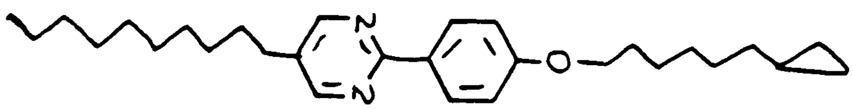

Phasenfolge: K 42 $S_c$ 60 $S_A$ 65 I

**Beispiel 17**

5-Heptyloxy-2-[4-(6-cyclopropyl-hexyl)oxy-phenyl]pyrimidin

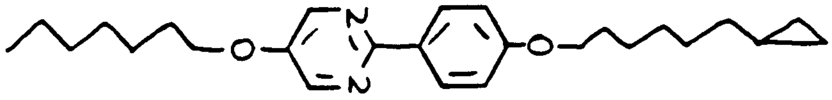

Phasenfolge: K 54 $S_c$ 72 $S_A$ 78 N 88 I

**Beispiel 18**

5-Octyloxy-2-[4-(6-cyclopropyl-hexyl)oxy-phenyl]pyrimidin

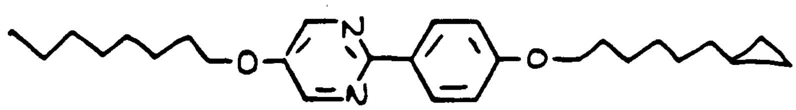

hat $S_c/S_A$-Übergang bei 81, $S_A$/N-Übergang bei 89 und Klärpunkt bei 92 °C.

**Beispiel 19**

5-(6-Cyclopropyl-hexyl)oxy-2-(4-nonyloxy-phenyl)pyrimidin

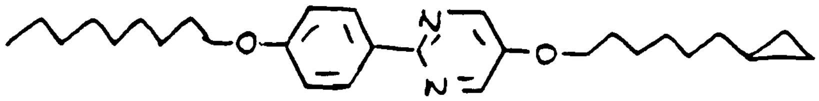

Phasenfolge: K 56,5 $S_c$ 79 $S_A$ 85 N 89,5 I

**Beispiel 20**

5-(6-Cyclopropyl-hexyl)oxy-2-(4-undecyloxy-phenyl)pyrimidin

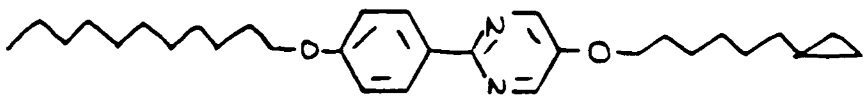

Phasenfolge: K 57,5 $S_c$ 76,5 $S_A$ 86,7 N 87 I

**Beispiel 21**

5-(6-Cyclopropyl-hexyl)oxy-2-(4-dodecyloxy-phenyl)pyrimidin

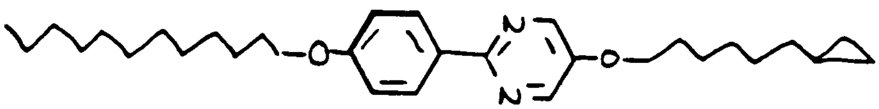

Phasenfolge: K 61 $S_c$ 77 $S_A$ 87 I

**Beispiel 22** (Vergleichsbeispiel)

trans-2-⟨9-[4-(5-Octyl-pyrimidin-2-yl)phenyloxy]nonyl⟩-cyclopropancarbonsäureethylester

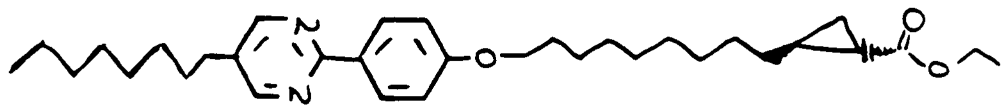

Phasenfolge: K [11 $S_c$ 36 $S_A$] 38 I

**Beispiel 23**

5-Octyl-2-[4-(7-cyclopropyl-heptyl)oxy-phenyl]pyrimidin

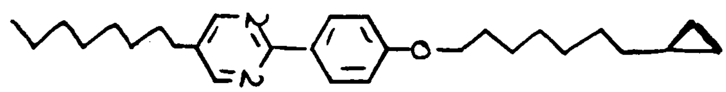

Phasenfolge: K 33 $S_c$ 45,5 $S_A$ 54,6 N 58,4 I

**Beispiel 24**

5-(7-Cyclopropyl-heptyl)oxy-2-(4-nonyloxy-phenyl)pyrimidin

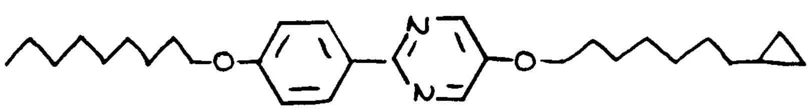

Phasenfolge: K 60 $S_c$ 87,9 $S_A$ 90,4 I

**Beispiel 25**

5-(7-Cyclopropyl-heptyl)oxy-2-(4-undecyloxy-phenyl)pyrimidin

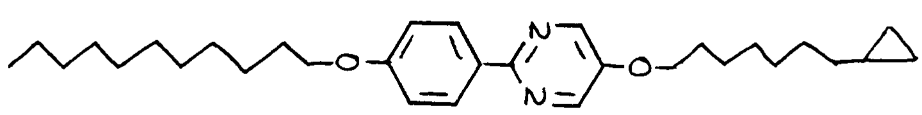

Phasenfolge: K 53,4 $S_c$ 87,6 $S_A$ 90,5 I

**Beispiel 26**

5-(7-Cyclopropyl-heptyl)oxy-2-(4-dodecyloxy-phenyl)pyrimidin

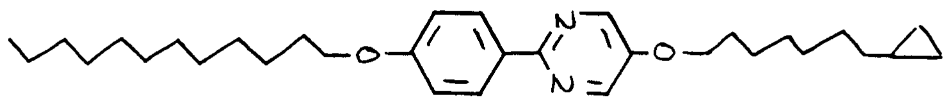

Phasenfolge: K 67,4 $S_c$ 88,1 $S_A$ 90,5 I

**Beispiel 27**

5-Octyloxy-2-[4-(7-cyclopropyl-heptyl)oxy-phenyl]pyrimidin

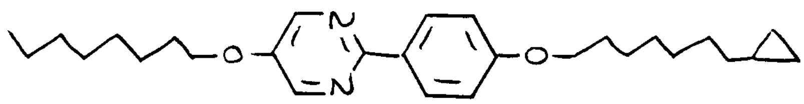

Phasenfolge: K 60 $S_c$ 78,2 $S_A$ 90 N 90,2 I

**Beispiel 28**

2-Octylthio-5-[4-(7-cyclopropyl-heptyl)oxy-phenyl]pyrimidin

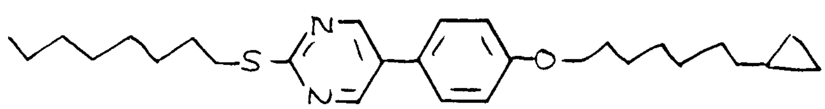

Phasenfolge: K [41 $S_3$ 42 $S_c$ 55,5] 61,6 $S_A$ 62,2 I

**Beispiel 29**

5-Octyl-2-[4-(11-cyclopropyl-5-oxa-undecyl)oxy-phenyl]pyrimidin

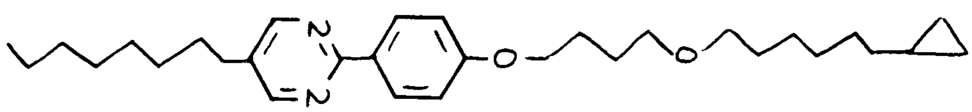

Phasenfolge: K [16 $S_c$ 25,6 N 31] 40,9 I

**Beispiel 30**

5-(9-Cyclopropyl-nonyl)oxy-2-[4-(trans-4-pentyl-cyclohexyl)carbonyloxy-phenyl]pyrimidin

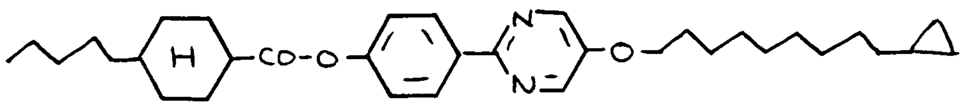

Phasenfolge: K [76 $S_2$ 84,3] 86,5 $S_c$ 129,2 N 179 I

**Beispiel 31**

5-(6-Cyclopropyl-5-oxa-hexyl)oxy-2-(4-nonyloxy-phenyl)pyrimidin

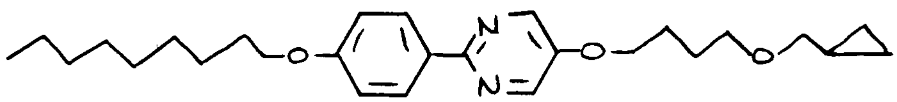

Phasenfolge: K [58 $S_c$ 58,4 N 72] 72,4 I

**Beispiel 32**

5-Octyl-2-[4-(6-cyclopropyl-5-oxa-hexyl)oxy-phenyl]pyrimidin

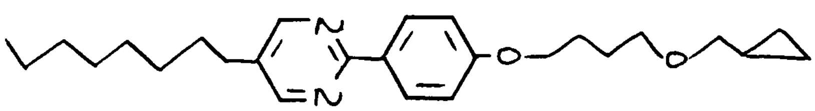

Phasenfolge: K [-4 $S_c$ 22] 28,5 N 40,2 I

**Beispiel 33**

5-(9-Cyclopropyl-nonyl)oxy-2-(4-undecyloxy-phenyl)pyrimidin

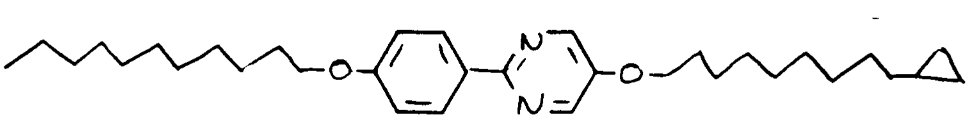

Phasenfolge: K 55 $S_c$ 94,2 I

**Beispiel 34**

5-(9-Cyclopropyl-nonyl)oxy-2-(4-dodecyloxy-phenyl)pyrimidin

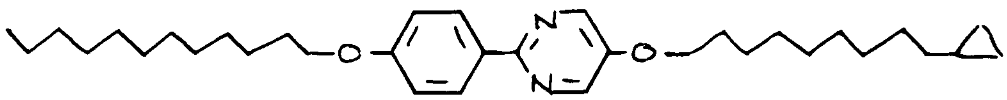

Phasenfolge: K 63 $S_c$ 94,2 I

**Beispiel 35**

5-Decyl-2-[4-(7-cyclopropyl-heptyl)oxy-phenyl]pyrimidin

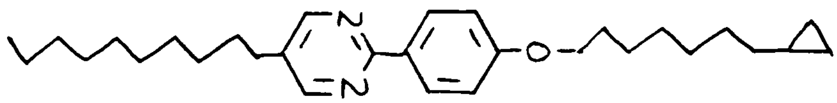

Phasenfolge: K 40,3 $S_c$ 61 $S_A$ 66,2 I

**Beispiel 36**

5-(7-Cyclopropyl-heptyl)oxy-2-(4-octyloxy-phenyl)pyrimidin

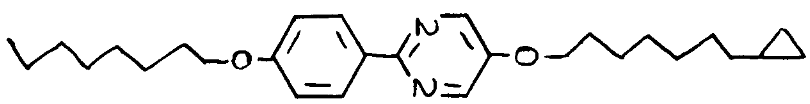

Phasenfolge: K 56,5 $S_c$ 89,1 $S_A$ 91,6 I

**Beispiel 37**

5-Dodecyl-2-[4-(11-cyclopropyl-undecyl)oxy-phenyl]pyrimidin

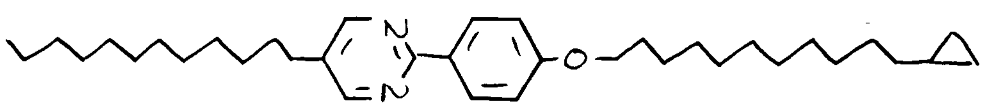

Phasenfolge: K 72 $S_c$ 95,3 I

**Beispiel 38**

5-Decyl-2-[4-(11-cyclopropyl-undecyl)oxy-phenyl]pyrimidin

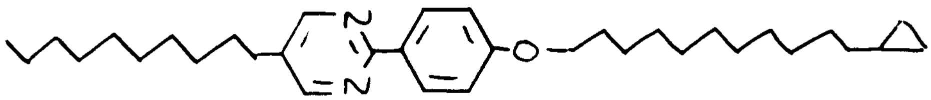

Phasenfolge: K 51,7 $S_c$ 65,6 $S_A$ 67,3 I

**Beispiel 39**

5-(7-Cyclopropyl-heptyl)oxy-2-(4-hexyloxy-phenyl)pyrimidin

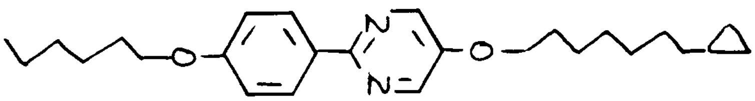

Phasenfolge: K 51,8 $S_c$ 86,5 $S_A$ 89,6 N 89,8 I

**Beispiel 40**

5-Octyl-2-[4-(8-cyclopropyl-octyl)oxy-phenyl]pyrimidin

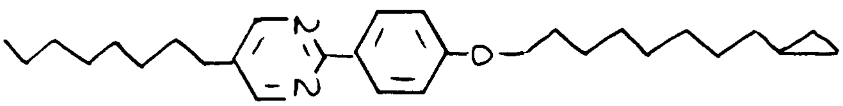

Phasenfolge: K 35 $S_c$ 51,5 $S_A$ 55,5 N 61,2 I

**Beispiel 41**

5-(8-Cyclopropyl-octyl)oxy-2-(4-nonyloxy-phenyl)pyrimidin

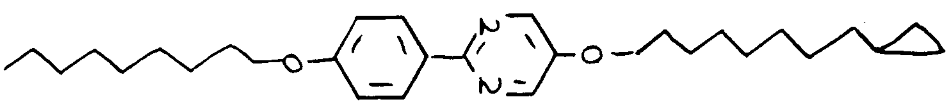

Phasenfolge: K 56,2 $S_c$ 91,8 $S_A$ 93 I

**Beispiel 42**

5-(8-Cyclopropyl-octyl)oxy-2-(4-undecyloxy-phenyl)pyrimidin

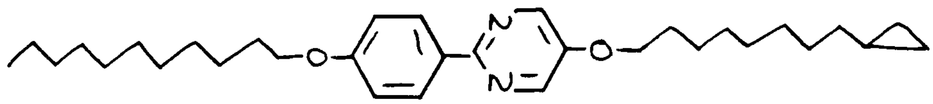

Phasenfolge: K 53,6 $S_c$92,3 $S_A$ 93,1 I

**Beispiel 43**

5-(8-Cyclopropyl-octyl)oxy-2-(4-dodecyloxy-phenyl)pyrimidin

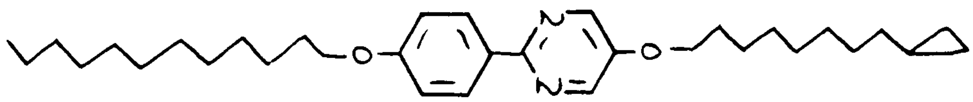

Phasenfolge: K 54,9 $S_c$ 92,3 $S_A$ 93 I

**Beispiel 44**

5-Octyloxy-2-[4-(8-cyclopropyl-octyl)oxy-phenyl]pyrimidin

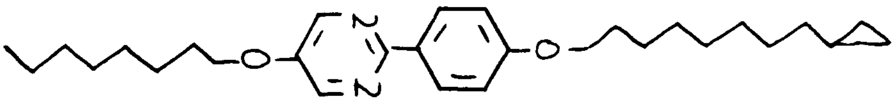

Phasenfolge: K 51 $S_c$ 79,2 $S_A$ 91 N 91,6 I

**Beispiel 45**

2-Octylthio-5-[4-(8-cyclopropyl-octyl)oxy-phenyl]pyrimidin

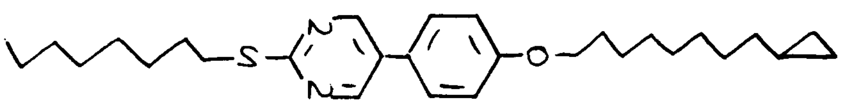

Phasenfolge: K [42,2 $S_c$ 59,5 $S_A$ 62,5] 64,7 I

**Beispiel 46**

5-(8-Cyclopropyl-octyl)oxy-2-(4-hexyloxy-phenyl)pyrimidin

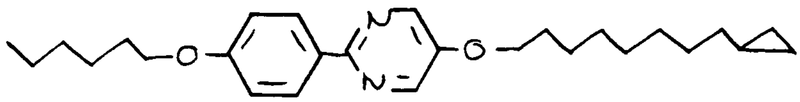

Phasenfolge: K 54,1 $S_c$ 88,2 $S_A$ 90,8 I

**Beispiel 47**

5-(8-Cyclopropyl-octyl)oxy-2-(4-octyloxy-phenyl)pyrimidin

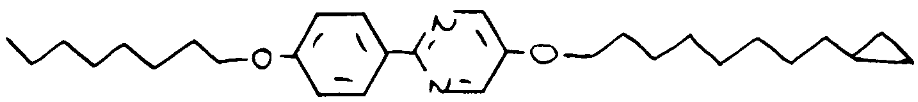

Phasenfolge: K 56,4 $S_c$ 91,7 $S_A$ 92,9 I

**Beispiel 48**

5-(11-Cyclopropyl-undecyl)oxy-2-(4-dodecyloxy-phenyl)pyrimidin

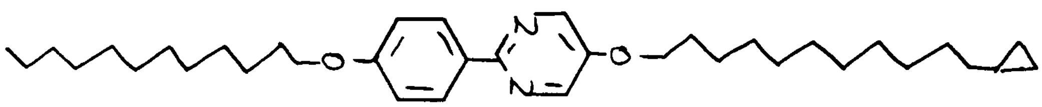

Phasenfolge: K 54,6 $S_c$ 73,8 I

**Beispiel 49**

3-(9-Cyclopropyl-nonyl)oxy-6-(4-octyloxy-phenyl)pyridazin

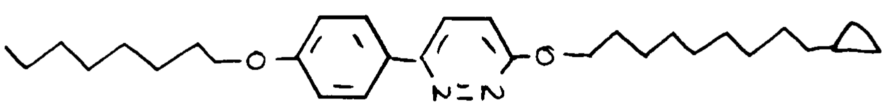

Phasenfolge: K [82 $S_c$ 95,7] 100 I

**Beispiel 50**

5-Decyl-2-[4-(8-cyclopropyl-octyl)oxy-phenyl]pyrimidin

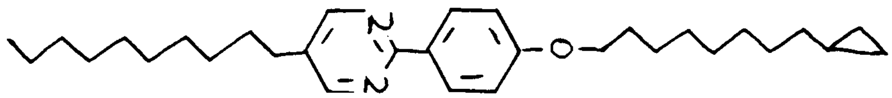

Phasenfolge: K 42,3 $S_c$ 62,5 $S_A$ 67,2 I

**Beispiel 51**

5-Octyloxy-2-[4-(cyclopropylmethyl)oxy-phenyl]pyrimidin

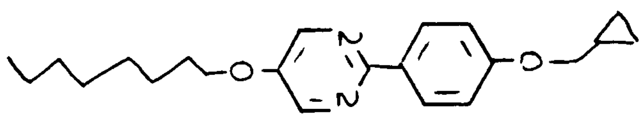

Phasenfolge: K 59 $S_c$ 62,8 $S_A$ 72,9 N 73,6 I

**Beispiel 52**

2-(9-Cyclopropyl-nonyl)oxy-5-[4-(9-cyclopropyl-nonyloxy)phenyl]pyrimidin

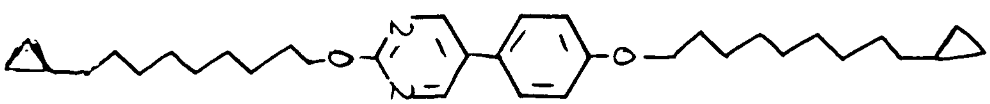

Phasenfolge: K [79,3 $S_c$ 79,5] 89,4 I

**Beispiel 53**

2-(9-Cyclopropyl-nonyl)oxy-5-(4-decyloxyphenyl)pyrimidin

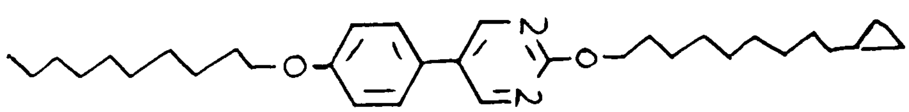

Phasenfolge: K [70 $S_3$ 70,55 $S_c$ 84 $S_A$ 87,6] 88 I

**Beispiel 54** (Vergleichsbeispiel)

trans-2-Hexyl-cyclopropancarbonsäure-[4-(2-octylthiopyrimidin-5-yl)]phenyl-ester

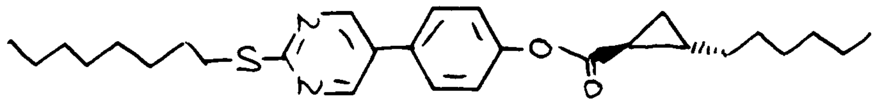

Phasenfolge: K 40,5 I

**Beispiel 55**

5-(8-Cyclopropyl-octyl)oxy-2-(4-decyloxy-phenyl)pyrimidin

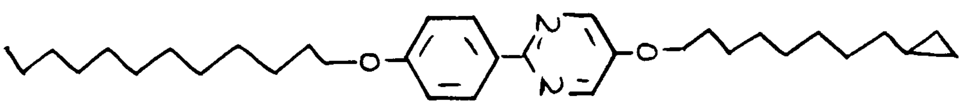

Phasenfolge: K 58,5 $S_c$ 91,6 $S_A$ 92 I

**Beispiel 56**

5-(8-Cyclopropyl-octyl)oxy-2-[4-butyloxy-phenyl)pyrimidin

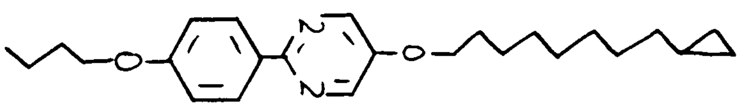

Phasenfolge: K 55,4 $S_c$ 81 $S_A$ 87,8 I

**Beispiel 57**

(R)-[4-⟨2-(9-Cyclopropyl-nonyl)oxy-pyrimidin-5-yl ⟩]phenyl-2,2-dimethyl-1,3-dioxolan-4-carbonsäureester

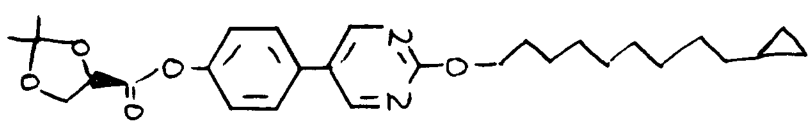

Phasenfolge: K 84 I $[\alpha]_D^{20}$ : + 5,46 (C = 2, $CH_2Cl_2$)

**Beispiel 58**

(2S,3S)-[4-⟨2-(9-Cyclopropyl-nonyl)oxy-pyrimidin-5-yl⟩]-phenyl-2-chlor-3-methyl-pentanoat

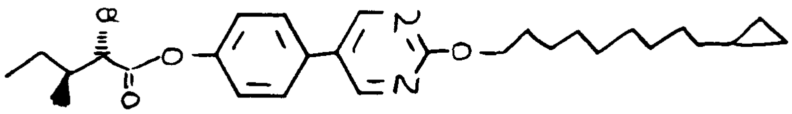

Phasenfolge: K 81 I $[\alpha]_D^{20}$ : + 1,2 (C = 2, $CH_2Cl_2$)

**Beispiel 59**

5-Octyl-2-[4-(4-cyclopropyl-butyl)oxy-phenyl]pyrimidin

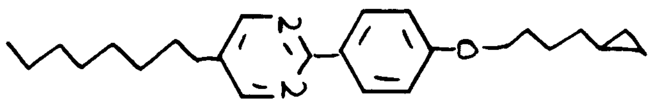

Phasenfolge: K [16 $S_c$ 37 $S_A$ 43,6] 45 N 56 I

**Beispiel 60**

5-Decyl-2-[4-(4-cyclopropyl-butyl)oxy-phenyl]pyrimidin

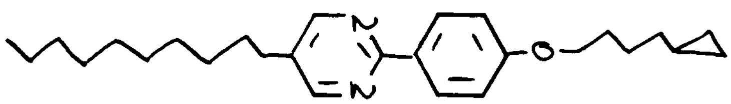

Phasenfolge: K [47 $S_c$ 48] 64 N 88 I

**Beispiel 61**

5-(4-Cyclopropyl-butyl)oxy-2-(4-hexyloxy-phenyl)pyrimidin

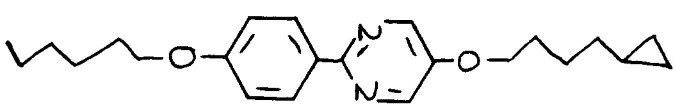

Phasenfolge: K [47 $S_c$ 48] 64 N 88 I

**Beispiel 62**

5-Octyl-2-[4-(5-cyclopropyl-pentyl)oxy-phenyl]pyrimidin

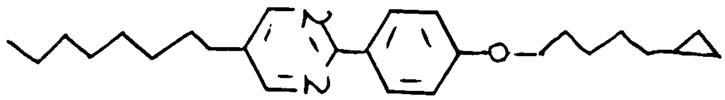

Phasenfolge: K [18 $S_c$ 34] 38 $S_A$ 51 N 54 I

**Beispiel 63**

5-Decyl-2-[4-(5-cyclopropyl-pentyl)oxy-phenyl]pyrimidin

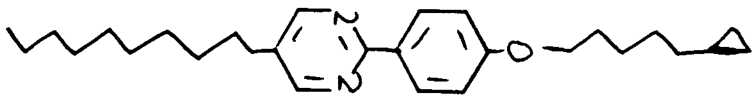

Phasenfolge: K 48 $S_c$ 53 $S_A$ 62 I

**Beispiel 64**

5-(5-Cyclopropyl-pentyl)oxy-2-(4-hexyloxy-phenyl)pyrimidin

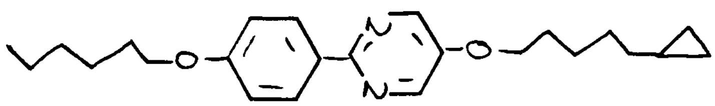

Phasenfolge: K 53 $S_c$ 73 $S_A$ 75 N 86 I

**Beispiel 65**

5-(7-Cyclopropyl-heptyl)oxy-2-(4-decyloxy-phenyl)pyrimidin

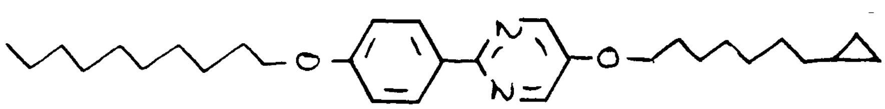

Phasenfolge: K 55,7 $S_c$ 90 $S_A$ 92,5 I

**Beispiel 66** (Vergleichsbeispiel)

trans-2-Hexyl-cyclopropylcarbonsäure-[4-(5-octyl-pyrimidin-2-yl)]phenyl-ester

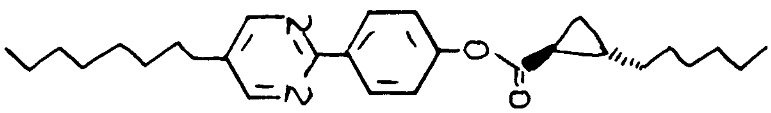

Phasenfolge: K 44 I

**Beispiel 67**

(2R,3R)-3-Propyl-oxiran-2-carbonsäure-[4-⟨2-(9-cyclopropylnonyl)oxy-pyrimidin-5-yl⟩]phenyl-ester

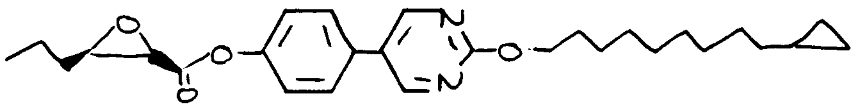

Phasenfolge: K 75 I $[\alpha]_D^{20}$ : - 9,6 (c = 2, $CH_2Cl_2$)

**Beispiel 68**

(2S)-2-Fluor-3-methyl-butansäure-[4-⟨2-(9-cyclopropylnonyloxy)-pyrimidin-5-yl⟩]phenyl-ester

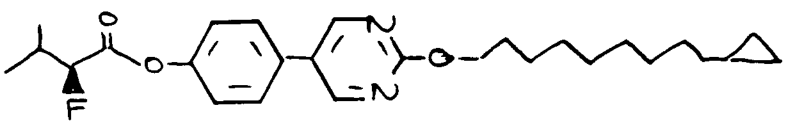

Phasenfolge: X [63 $S_A$ 64] 78 I $[\alpha]_D^{20}$ : - 1,0 (c = 2, $CH_2Cl_2$)

**Beispiel 69**

2-(4-Hexyloxy-phenyl)-5-[4-(6-cyclopropyl-hexyloxy)phenyl]pyrimidin

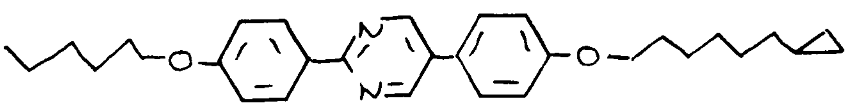

Phasenfolge: X [77 $S_4$ 103] 110 $S_3$ 129 $S_c$ 189 $S_A$ 198 I

**Beispiel 70**

5-(4-Cyclopropyl-butyloxy)-2-[4-(5-oxa-nonyloxy)phenyl]pyrimidin

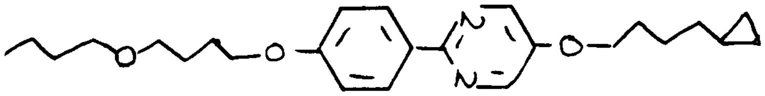

Phasenfolge: K 42 $S_c$ 45 $S_A$ 47 N 64 I

**Beispiel 71**

5-(5-Cyclopropyl-[pentyloxy)-2-[4-(5-oxa-nonyloxy)phenyl]pyrimidin

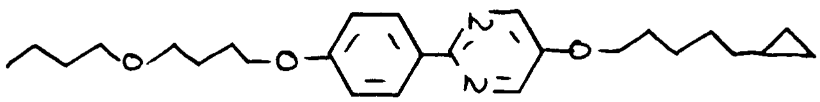

Phasenfolge: X 39 $S_c$ 63 $S_A$ 65 N 67 I

**Beispiel 72**

5-(6-Cyclopropyl-hexyloxy)-2-[4-(5-oxa-nonyloxy)phenyl]pyrimidin

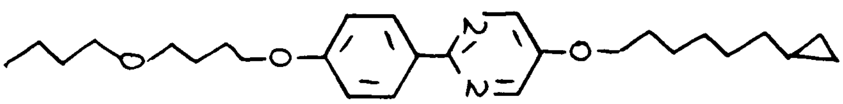

Phasenfolge: X 46 $S_c$ 66 $S_A$ 67 N 69 I

**Beispiel 73**

5-(7-Cyclopropyl-heptyloxy)-2-[4-(5-oxa-nonyloxy)phenyl]pyrimidin

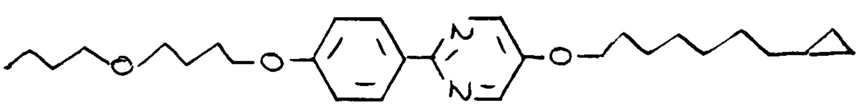

Phasenfolge: K 43 $S_c$ 73 I

**Beispiel 74**

5-Cyclopropylmethyloxy-2-(4-octyloxyphenyl)pyrimidin

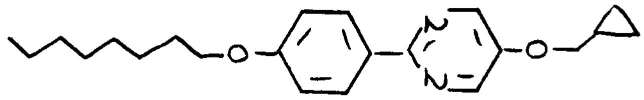

Phasenfolge: K 63 N 64 I

**Beispiel 75**

5-Octyl-2-[4-(6-cyclopropyl-hexyloxy)phenyl]pyrimidin

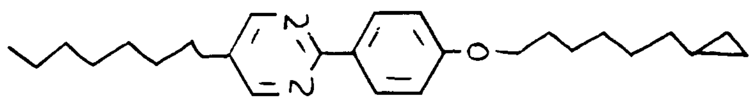

Phasenfolge: K 37 $S_c$ 46 $S_A$ 50 N 59 I

**Beispiel 76**

5-Octyloxy-2-[4-(6-cyclopropyl-hexyloxy)phenyl]pyrimidin

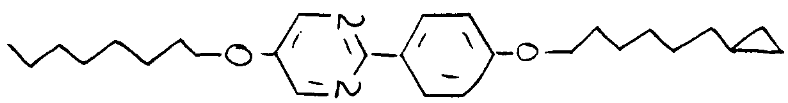

Phasenfolge: K 56 $S_c$ 80 $S_A$ 88 N 91 I

**Beispiel 77**

5-(6-Cyclopropyl-hexyloxy)-2-(4-octyloxyphenyl)pyrimidin

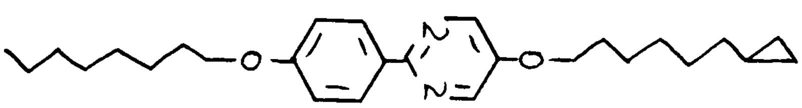

Phasenfolge: K 56 $S_c$ 78 $S_A$ 84 N 89 I

**Beispiel 78**

2-[4-(7-Cyclopropyl-heptyloxy)phenyl]-5-octyl-pyridin

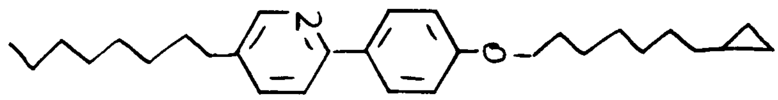

Phasenfolge: K 49 $S_2$ 63 $S_c$ 72 I

**Beispiel 79**

2-[4-(11-Cyclopropyl-undecyloxy)phenyl]-5-octyl-pyridin

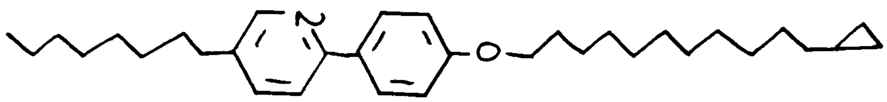

Phasenfolge: K 57 $S_2$ 63 $S_c$ 72 I

**Beispiel 80**

2-(trans-4-Pentyl-cyclohexyl)-5-[4-(11-cyclopropyl-undecyloxy)-phenyl]-1,3,4-thiadiazol

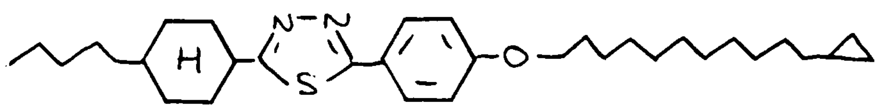

Phasenfolge: K 111 $S_c$ 113 $S_A$ 156 N 157 I

**Beispiel 81**

7-Cyclopropyl-heptansäure-[4-(5-octyl-pyrimidin-2-yl)]phenyl-ester

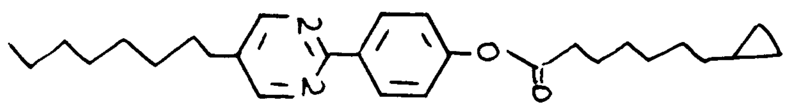

Phasenfolge: K [36 $S_c$ 40,5 $S_A$ 44 N 46] 51 I

**Beispiel 82**

7-Cyclopropyl-heptansäure-[4-(5-decyl-pyrimidin-2-yl)]phenyl-ester

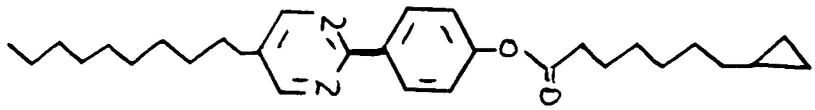

Phasenfolge: K 48 $S_c$ 57 I

**Beispiel 83**

7-Cyclopropyl-heptansäure-[4-(5-octyloxy-pyrimidin-2-yl)]phenyl-ester

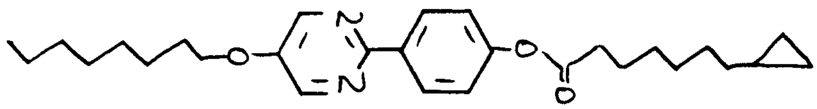

Phasenfolge: K 62 $S_c$ 77 $S_A$ 84 N 84,3 I

**Anwendungsbeispiel 1**

Eine Mischung bestehend aus 55 mol-% Verbindung nach Beispiel 6 und 45 mol-% Verbindung nach Beispiel 3 zeigt die Phasenfolge K 31 $S_c$ 58 $S_A$ 64 I

**Anwendungsbeispiel 2**

Eine Mischung bestehend aus 67 mol-% Verbindung nach Beispiel 6 und 33 mol-% Verbindung nach Beispiel 11 zeigt die Phasenfolge K 35 $S_c$ 74 $S_A$ 78 I

**Anwendungsbeispiel 3**

Eine Mischung bestehend aus 20 mol-% der Verbindung nach Beispiel 6, 25,15 mol-% (der jeweils bekannten Mischungskomponente) 5-Octyloxy-2-(4-decyloxy-phenyl)-pyrimidin, 11 mol-% 5-Octyloxy-2-(4-octyloxy-phenyl)-pyrimidin, 20 mol-% 5-Octyloxy-2-(4-hexyloxy-phenyl)-pyrimidin und 23,85 mol-% 5-Octyloxy-2-(4-butyloxy-phenyl)pyrimidin zeigt die Phasenfolge K 11,5 $S_c$ 72 $S_A$ 88 N 90 I.

**Anwendungsbeispiel 4**

Eine Mischung aus 60 mol-% Verbindung nach Beispiel 56 und 40 mol-% Verbindung nach Beispiel 55 zeigt die Phasenfolge K 34 $S_c$ 82 $S_A$ 90 I.

**Anwendungsbeispiel 5**

Eine Mischung aus 62 mol-% Verbindung nach Beispiel 43 und 38 mol-% Verbindung nach Beispiel 37 zeigt die Phasenfolge K 48 $S_c$ 93 I.

**Anwendungsbeispiel 6**

Eine Mischung aus 57 mol-% Verbindung nach Beispiel 43 und 43 mol-% Verbindung nach Beispiel 65 zeigt die Phasenfolge K 36 $S_c$ 90 $S_A$ 92 I.

**Anwendungsbeispiel 7**

Eine Mischung aus 60 mol-% Verbindung nach Beispiel 27 und 40 mol-% Verbindung nach Beispiel 47 zeigt die Phasenfolge K 73 $S_c$ 85 $S_A$ 91 I.

**Anwendungsbeispiel 8**

Eine Mischung aus 45 mol-% Verbindung nach Beispiel 24 und 55 mol-% Verbindung nach Beispiel 71 zeigt die Phasenfolge K 25 $S_c$ 70 $S_A$ 77 N 79 I.

**Anwendungsbeispiel 9**

Eine Mischung aus 30 mol-% Verbindung nach Beispiel 65 und 70 mol-% Verbindung nach Beispiel 71 zeigt die Phasenfolge K 25 $S_c$ 70 $S_A$ 73 N 75 I.

**Anwendungsbeispiel 10**

Eine Mischung aus 40 mol-% Verbindung nach Beispiel 76 und 60 mol-% Verbindung nach Beispiel 56 zeigt die Phasenfolge K 35 $S_c$ 81 $S_A$ 90 I.

**Anwendungsbeispiel 11**

Eine Mischung aus 65 mol-% Verbindung nach Beispiel 71 und 35 mol-% Verbindung nach Beispiel 56 zeigt die Phasenfolge K 17 $S_c$ 69 $S_A$ 73 N 74 I.

Die Anwendungsbeispiele 10 und 11 weisen gegenüber einer Vergleichsmischung (binäre Mischung mit Verbindungen vergleichbarer Kettenlängen ohne Cyclopropyl-Rest) aus 40 mol-% 5-Octyloxy-2-(4-octyloxy-phenyl)pyrimidin und 60 mol-% 5-Octyloxy-2-(4-hexyloxy-phenyl)pyrimidin mit der Phasenfolge K 39 $S_c$ 90 $S_A$ 98 N 100 I sowohl einen niedrigeren Schmelzpunkt als auch eine größere Schmelzpunktdepression auf.

**Anwendungsbeispiel 12**

Eine Mischung aus 50 mol-% 5-Dodecyloxy-2-(4-octyloxy-phenyl)pyrimidin und 50 mol-% der Verbindung nach Beispiel 24 weist die Phasenfolge K 33 $S_c$ 96 I auf. Gegenüber den Vergleichsmischungen (binäre Mischungen vergleichbarer Kettenlängen ohne Cyclopropyl-Rest) aus 39 mol-% 5-Decyloxy-2-(4-octyloxy-phenyl)pyrimidin und 61 mol-% 5-Octyloxy-2-(4-decyloxy-phenyl)pyrimidin mit der Phasenfolge K 39 $S_c$ 94 $S_A$ 100 I oder aus 60 mol-% 5-Octyloxy-2-(4-decyloxy-phenyl)pyrimidin und 40 mol-% 5-Octyloxy-2-(4-dodecyloxy-phenyl)pyrimidin mit der Phasenfolge K 40 $S_c$ 86 $S_A$ 97 I sowohl einen niedrigeren Schmelzpunkt als auch eine größere Schmelzpunktdepression auf.

**Anwendungsbeispiel 13**

Eine Mischung aus
30 mol-% 5-Octyloxy-2-(4-ethyloxy-phenyl)pyrimidin
6 mol-% 5-Dodecyloxy-2-(4-butyloxy-phenyl)pyrimidin
15 mol-% Verbindung nach Beispiel 24
19 mol-% Verbindung nach Beispiel 71
20 mol-% Verbindung nach Beispiel 56
10 mol-% Verbindung nach Beispiel 43
weist die Phasenfolge K 8 $S_c$ 68 $S_A$ 85 N 87 auf.

**Anwendungsbeispiel 14**

Eine ferroelektrische Multikomponentenmischung die 10 mol-% der Verbindung nach Beispiel 62 in (R)Felix 001*) enthält, weist folgende Phasenfolge auf K -5 $S_c^*$ 72 $S_A^*$ 78 N* 93 I .

*) (C. Escher, H.-R. Dübal, W. Hemmerling, I. Müller, D. Ohlendorf und R. Wingen, vorgetragen auf "1st International Symposium on Ferroelectric Liquid Crystals, Arcachon, Bordeaux-France, 21.-23. Sept. 1987", handelsübliche Mischung der Hoechst Aktiengesellschaft mit der Phasenfolge K -7 $S_C^*$ 79 $S_A^*$ 83 N* 99 I)

Die Mischung ist nach konventionellen Methoden gut orientierbar und ist bistabil. Bei 25 °C zeigt die Mischung eine spontane Polarisation von -5,8 $nC/cm^2$ und weist folgende Schaltzeiten auf:
$\tau_{0-90}$ = 213 $\mu s$
$\tau_{10-90}$ = 90 $\mu s$
Die Viskosität der Mischung liegt bei 65 mPas und der doppelte effektive Tiltwinkel ist 18°.

**Anwendungsbeispiel 15**

Eine ferroelektrische Mischung
aus 85,5 mol-% der Mischung aus Anwendungsbeispiel 13
mit 9,5 mol-%
trans-4-Pentyl-cyclohexancarbonsäure-[4-(5-decyl-pyrimidin-2-yl)]phenyl-ester
und 5 mol-%
4-[2-((S)-7-Methyl-nonyloxy)primidin-5-yl]-phenyl-(2S,3S)-2-chlor-3-methyl-pentansäureester
weist die Phasenfolge K 5 $S_c^*$ 72 $S_A^*$ 83 N* 88 I auf.
Die Mischung ist nach konventionellen Methoden gut orientierbar und ist bistabil. Bei 25 °C zeigt die Mischung eine spontane Polarisation von -8,2 $nC/cm^2$ und weist folgende Schaltzeiten auf:
$\tau_{0-90}$ = 139 $\mu s$
$\tau_{10-90}$ = 66 $\mu s$
Die Viskosität der Mischung liegt bei 280 mPas und der doppelte effektive Tiltwinkel beträgt 17°.

**Patentansprüche**

**1.** Flüssigkristalline Cyclopropylalkyl- oder -alkenyl-Heterocyclen der allgemeinen Formel (I)

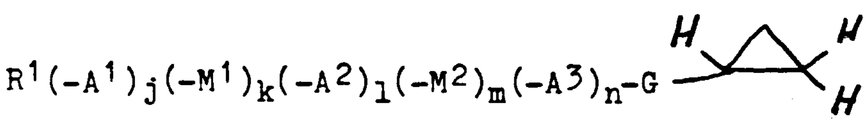

(I)

in der bedeuten:

R[1] geradkettiges oder verzweigtes (mit oder ohne asymmetrisches C-Atom) Alkyl oder Alkenyl mit 2 bis 16 C-Atomen, wobei auch eine oder Zwei nicht-benachbarte $-CH_2-$ Gruppen durch -O-, -S-, -CO-, -CO-O-, -O-CO- oder -O-CO-O-ersetzt sein können und wobei auch H durch F ersetzt sein kann,
oder einer der nachfolgenden Reste

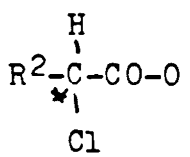
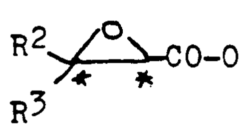
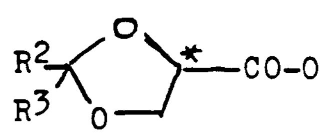

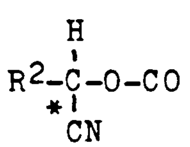
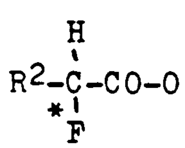
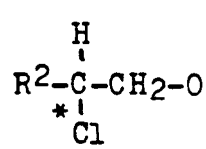

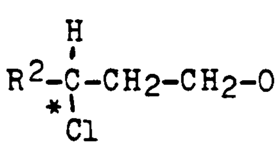
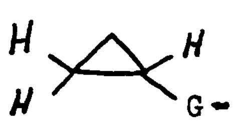

| | |
|---|---|
| $A^1$,$A^2$,$A^3$ | gleich oder verschieden 1,4-Phenylen, trans-1,4-Cyclohexylen, Pyrazin-2,5-diyl, Pyridazin-3,6-diyl, Pyridin-2,5-diyl, Pyrimidin-2,5-diyl, (1,3,4)-Thiadiazol-2,5-diyl |
| $M^1$,$M^2$ | gleich oder verschieden CO-O, O-CO, CO-S, S-CO, $CH_2$-O, O-$CH_2$ |
| G | geradkettiges oder verzweigtes Alkylen mit 1 bis 16 C-Atomen oder Alkenylen mit 2 bis 16 C-Atomen, bei dem auch eine oder zwei nicht-benachbarte -$CH_2$-Gruppen durch -O-, -S-, -O-CO-, -CO-O-, -S-CO- oder -CO-S- ersetzt sein können |
| $R^2$,$R^3$,$R^4$ | H oder geradkettiges oder verzweigtes Alkyl mit 1 bis 16 oder Alkenyl mit 2 bis 16 C-Atomen, bei dem auch eine -$CH_2$-Gruppe durch -O-, -CO-O- oder -O-CO- ersetzt sein kann |
| j,k,l,m,n | Null oder 1 |

mit folgenden Maßgaben: a) j+l+n=2 oder 3, b) eine der Gruppierungen $A^1$, $A^2$, $A^3$ ist nicht 1,4-Phenylen oder trans-1,4-Cyclohexylen.

**2.** Ausführungsform nach Anspruch 1, dadurch gekennzeichnet, daß in der allgemeinen Formel (I) die Gruppierung $(-A^1)_j(-M^1)_k(-A^2)_l(-M^2)_m(-A^3)_n$- bedeutet:

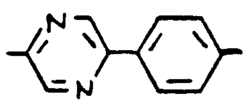, 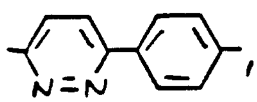, 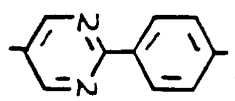, 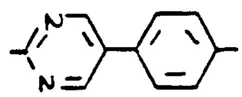,

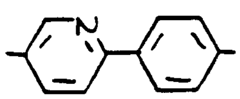, 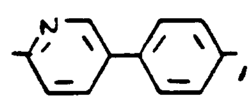, 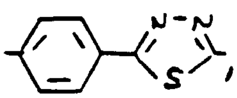, 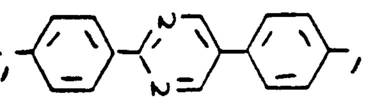,

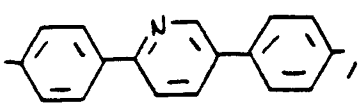, 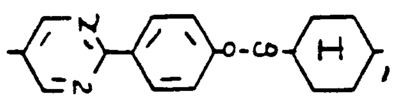, 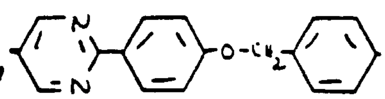

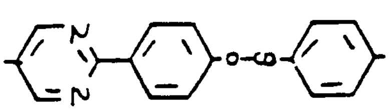, 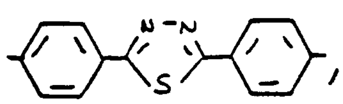, 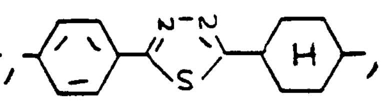,

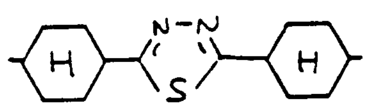, 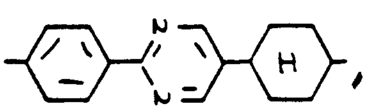, 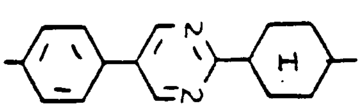

**3.** Verfahren zur Herstellung der Cyclopropylalkyl- oder -alkenyl-Heterocyclen der allgemeinen Formel (I) nach Anspruch 1 oder 2, dadurch gekennzeichnet, daß mesogene monofunktionell-reaktionsfähige Grundkörper der allgemeinen Formel (II) mit monofunktionell-reaktionsfähigen Cyclopropylalkyl-Verbindungen der allgemeinen Formel (III) umgesetzt werden,

$R^1(-A^1)_j(-M^1)_k(-A^2)_l(-M^2)_m(-A^3)_n$-X (II)

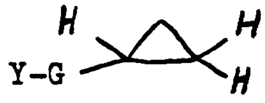

(III)

wobei X=OH, O-Alkali, COOH oder COO-Alkali und Y bei der Reaktion abgehende Substituenten wie H, OH, Alkali, Halogen, Toluolsulfonyloxy oder Methylsulfonyloxy bedeuten.

**4.** Verwendung der Cyclopropylalkyl- oder -alkenyl-Heterocyclen der allgemeinen Formel (I) nach Anspruch 1 oder 2 als Komponente in Flüssigkristallmischungen.

**5.** Verwendung nach Anspruch 4, dadurch gekennzeichnet, daß die Flüssigkristallmischung nematisch ist.

**6.** Verwendung nach Anspruch 4, dadurch gekennzeichnet, daß die Flüssigkristallmischung smektisch ist.

**7.** Flüssigkristalline Mischung enthaltend mindestens einen Cyclopropylalkyl- oder -alkenyl-Heterocyclus der allgemeinen Formel (I) nach Anspruch 1 oder 2.

**8.** Flektrooptisches Bauteil enthaltend eine flüssigkristalline Mischung nach Anspruch 7.

## Claims

**1.** A liquid-crystalline cyclopropylalkyl- or -alkenyl-heterocyclic compound of the formula (I)

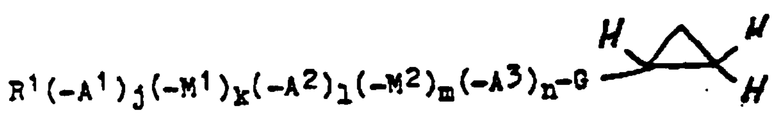

(I)

in which:

| | |
|---|---|
| $R^1$ | is straight-chain or branched (with or without an asymmetrical carbon atom) alkyl or alkenyl having 2 to 16 carbon atoms, in which, in addition, one or two non-adjacent $-CH_2-$ groups may be replaced by -O-, -S-, -CO-, -CO-O-, -O-CO- or -O-CO-O-, and in which, in addition, H may be replaced by F, or is one of the following radicals |

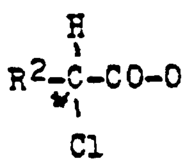
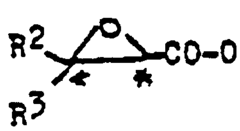
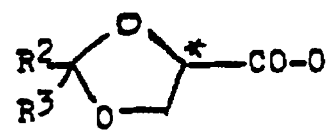

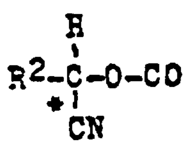
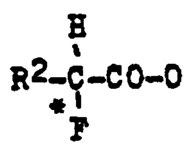
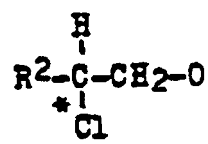

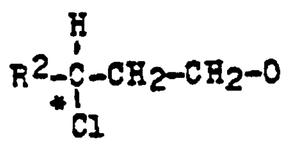
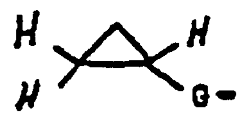

| | |
|---|---|
| $A^1$, $A^2$ and $A^3$ | are identical or different and are 1,4-phenylene, trans-1,4-cyclohexylene, pyrazine-2,5-diyl, pyridazine-3,6-diyl, pyridine-2,5-diyl, pyrimidine-2,5-diyl or 1,3,4-thiadiazole-2,5-diyl, |
| $M^1$ and $M^2$ | are identical or different and are CO-O, O-CO, CO-S, S-CO, $CH_2$-O or O-$CH_2$, |
| G | is straight-chain or branched alkylene having 1 to 16 carbon atoms or alkenylene having 2 to 16 carbon atoms, in which, in addition, one or two non-adjacent $-CH_2-$ groups may be replaced by -O-, -S-, -O-CO-, -CO-O-, -S-CO- or -CO-S-, |
| $R^2$, $R^3$ and $R^4$ | are H or straight-chain or branched alkyl having 1 to 16 or alkenyl having 2 to 16 carbon atoms, in which, in addition, one $-CH_2-$ group may be replaced by -O-, -CO-O- or -O-CO-, |
| j,k,l,m and n | are zero or 1, |

with the following provisos: a) $j+l+n = 2$ or 3, b) one of the groups $A^1$, $A^2$ and $A^3$ is not 1,4-phenylene

or trans-1,4-cyclohexylene.

**2.** An embodiment as claimed in claim 1, wherein, in the formula (I), the $(-A^1)_j(-M^1)_k(-A^2)_l(-M^2)_m(-A^3)_n$-group is:

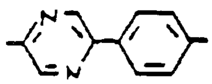, 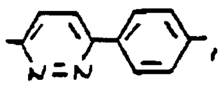, 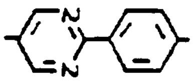, 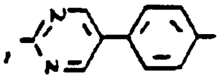,

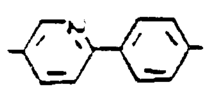, 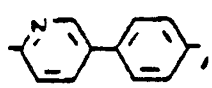, 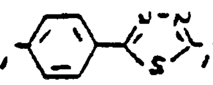, 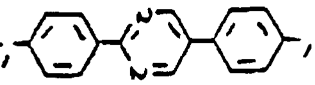,

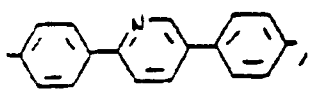, 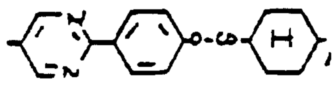, 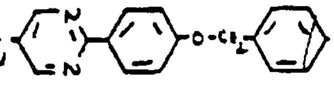

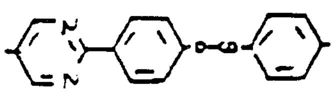, 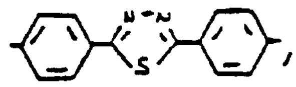, 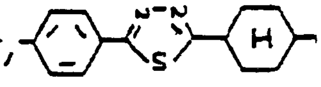

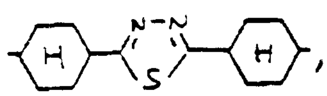, 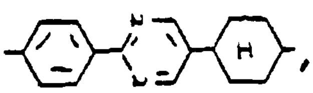, 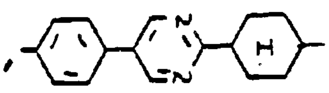

**3.** A process for the preparation of a cyclopropylalkyl-or -alkenyl-heterocyclic compound of the formula (I) as claimed in claim 1 or 2, which comprises reacting a mesogenic, monofunctionally reactive parent structure of the formula (II) with a monofunctionally reactive cyclopropylalkyl compound of the formula (III)

$R^1(-A^1)_j(-M^1)_k(-A^2)_l(-M^2)_m(-A^3)_n$-X (II)

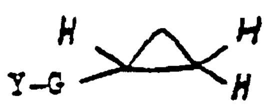

(III)

where X is OH, O-alkali metal, COOH or COO-alkali metal, and Y is a substituent which leaves during the reaction, such as H, OH, alkali metal, halogen, toluenesulfonyloxy or methylsulfonyloxy.

**4.** The use of a cyclopropylalkyl or -alkenyl-heterocyclic compound of the formula (I) as claimed in claim 1 or 2 as a component of liquid-crystal mixtures.

**5.** The use as claimed in claim 4, wherein the liquid-crystal mixture is nematic.

**6.** The use as claimed in claim 4, wherein the liquid-crystal mixture is smectic.

**7.** A liquid-crystalline mixture containing at least one cyclopropylalkyl- or -alkenyl-heterocyclic compound of the formula (I) as claimed in claim 1 or 2.

**8.** An electro-optical component containing a liquid-crystalline mixture as claimed in claim 7.

## Revendications

**1.** Composés cyclopropylalkyl- ou alcényl-hétéro-cycliques en cristaux liquides, de forlume générale I ci-dessous

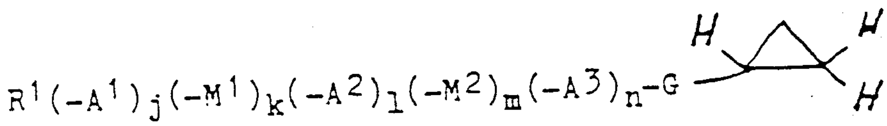

(I)

dans laquelle

$R^1$ représente un alkyle ou un alcényle linéaire ou ramifié avec ou sans atome de carbone asymétrique, ayant de 2 à 16 atomes de carbone, un ou deux groupes $-CH_2-$ non-voisins pouvant être aussi remplacés par -O-, -S-, -CO-, -CO-O-, -O-CO- ou -O-CO-O- et l'hydrogène pouvant être également remplacé par du fluor, ou bien l'un des radicaux suivants

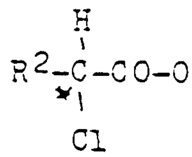
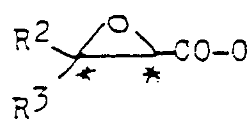
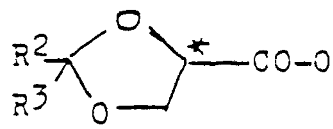
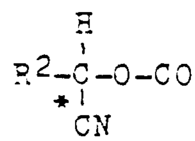
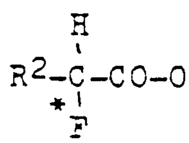
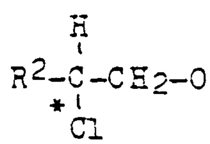
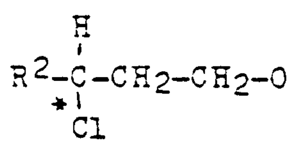
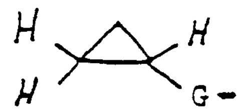

$A^1$, $A^2$ et $A^3$ qui peuvent être identiques ou différents les uns des autres, représentent chacun un groupe 1,4-phénylène, trans-1,4-cyclohexylène, pyrazine-2,5-diyle, pyridazine-3,6-diyle, pyridine-2,5-diyle, pyrimidine-2,5-diyle ou (1,3,4)-thiadiazole-2,5-diyle,

$M^1$ et $M^2$ qui peuvent être également identiques ou différents l'un de l'autre, représentent chacun un groupe CO-O, O-CO, CO-S, S-CO, $CH_2$-O ou $CH_2$,

G désigne un alkylène ayant de 1 à 16 atomes de carbone ou un alcénylène ayant de 2 à 16 atomes de carbone, linéaires ou ramifiés, un ou deux groupes $-CH_2-$ non-voisins pouvant être également remplacé par -O-, -S-, -O-CO-, -CO-O-, -S-CO- ou -CO-S-,

$R^2$, $R^3$ et $R^4$ étant l'hydrogène ou bien des alkyles avec de 1 à 16 atomes de carbone ou des alcényles avec de 2 à 16 atomes de carbone, linéaires ou ramifiés, dont aussi un groupe $-CH_2-$ peut être remplacé par -O-, -CO-O- ou -O-CO-, et

j, k, l, m et n sont chacun le nombre 0 ou 1,

avec les conditions que : a) la somme j + l + n soit égale à 2 ou 3, et b) l'un des groupes $A^1$, $A^2$ et $A^3$ ne soit pas un groupe 1,4-phénylène ou trans-1,4-cyclohexylène.

**2.** Composés selon la revendication 1, caractérisés en ce que le groupe $(-A^1)j(-M^1)k(-A^2)l(-M^2)m(-A^3)n-$ est l'un des suivants :

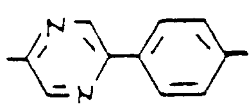, 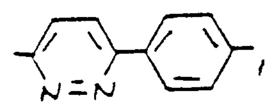, 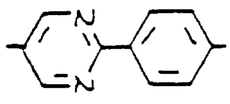, 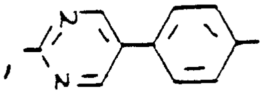,

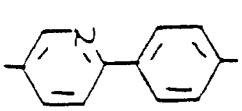, 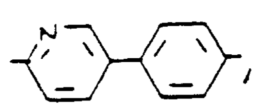, 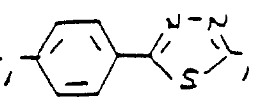, 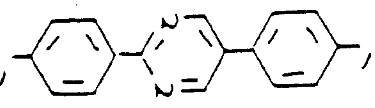,

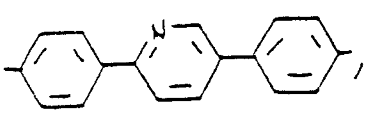, 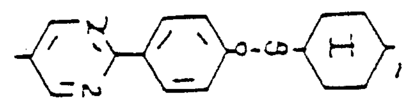, 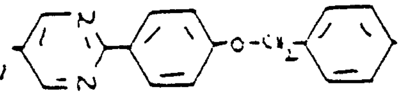

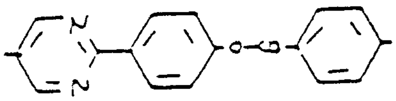, 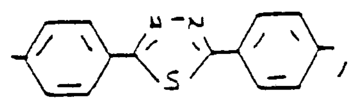, 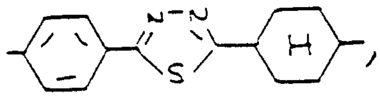,

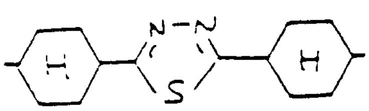, 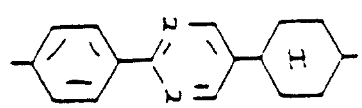, 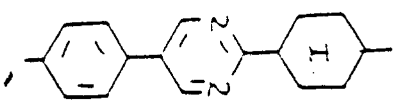

**3.** Procédé de préparation des composés hétéro-cycliques de formule I selon la revendication 1 ou 2, procédé caractérisé en ce que l'on fait réagir des matières de base mésogènes pouvant donner lieu à une réaction monofonctionnelle, de formule II, avec des composés cyclopropylalkyliques également capables de réactions monofonctionnelles, de formule III

$$R^1(-A^1)_j(-M^1)_k(-A^2)_l(-M^2)_m(-A^3)_n-X \qquad (II)$$

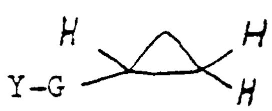

(III)

X désignant un groupe OH, O-métal alcalin, COOH ou COO-métal alcalin et Y un substituant qui s'élimine par la réaction, tel que H, OH, un métal alcalin, un halogène, un groupe toluène-sulfonyloxy ou méthylsulfonyloxy.

**4.** L'emploi des composés hétérocycliques de formule I selon la revendication 1 ou 2 comme composants de mélanges de cristaux liquides.

**5.** Emploi selon la revendication 4, caractérisé en ce que le mélange de cristaux liquides est de nature nématique.

**6.** Emploi selon la revendication 4, caractérisé en ce que le mélange de cristaux liquides est de nature smectique.

**7.** Mélange de cristaux liquides qui comprend un ou plusieurs composés hétérocycliques de formule I selon la revendication 1 ou 2.

**8.** Eléments électro-optiques comprenant un mélange de cristaux liquides selon la revendication 7.